# EUROPEAN PATENT APPLICATION

(11) **EP 4 485 052 A1**
(43) Date of publication of application: **01.01.2025**
(21) Application number: 23759958.4
(22) Date of filing: 21.02.2023
(51) Int. Cl.: G02C 7/04

(54) **OCULAR LENS, DESIGN METHOD AND MANUFACTURING METHOD FOR SAME, AND OCULAR LENS SET**

(30) Priority: 24.02.2022 JP 2022026362
(71) Applicant: HOYA CORPORATION, Shinjuku-ku Tokyo 160-8347 (JP)
(72) Inventor: SHIMOJO, Akira, Tokyo 160-8347 (JP)
(74) Representative: Betten & Resch
(86) International application number: PCT/JP2023/006143
(87) International publication number: WO 2023/162957

(57) **Abstract**

There is provided an ophthalmic lens 6 including an optical portion 4 that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens having a front surface arranged on an object side and a rear surface opposed to the front surface, wherein the front surface is a composite surface including a progressive surface and a toric surface, and a cylinder power of the near-vision portion is equal to an astigmatism correction power set in the near-vision portion, and a cylinder power of the far-vision portion is equal to an astigmatism correction power set in the far-vision portion. Its related art is also provided.

## Description

### Technical Field

The present invention relates to an ophthalmic lens, a design method of the same, a manufacturing method of the same, and an ophthalmic lens set.

### Description of Related Art

As an ophthalmic lens, for example, a contact lens, an intraocular lens, etc., are known (in this specification, a spectacle lens is excluded as an ophthalmic lens). For example, contact lenses include multifocal contact lenses (multifocal lenses) each ensuring both of a near-vision power for seeing a near distance and a far-vision power for seeing a far distance with a single lens. An example configuration of such a multifocal lens is a configuration in which a near-vision portion having a near-vision power is arranged at the center of the lens, an intermediate portion in which the power changes is arranged in a ring shape along an outer edge of the near-vision portion, and a far-vision portion having a far-vision power is arranged in a ring shape along an outer edge of the intermediate portion (e.g., FIG. 1 and FIG. 4 of Patent Document 1). Conversely, a configuration is also known in which a far-vision portion having a far-vision power is arranged at the center of the lens, an intermediate portion in which the power changes is arranged in a ring shape along an outer edge of the far-vision portion, and a near-vision portion having a near-vision power is arranged in a ring shape along an outer edge of the intermediate portion (e.g., FIG. 14 of Patent Document 2).

### Prior art document

### Patent document

[Patent Document 1] Japanese Unexamined Patent Application Publication (Translation of PCT Application) No. 2006-505011
[Patent Document 2] WO 2006/129707

### Summary of the Invention

### Problem to be solved by the Invention

Before describing a problem to be solved by the present invention, an optical portion will be described. Note that the following describes, merely as an example, a multifocal contact lens (a multifocal lens, also simply referred to as a "lens") in which a near-vision portion having a near-vision power is arranged at the center of the lens, an intermediate portion in which the power changes is arranged in a ring shape along an outer edge of the near-vision portion, and a far-vision portion having a far-vision power is arranged in a ring shape along an outer edge of the intermediate portion.

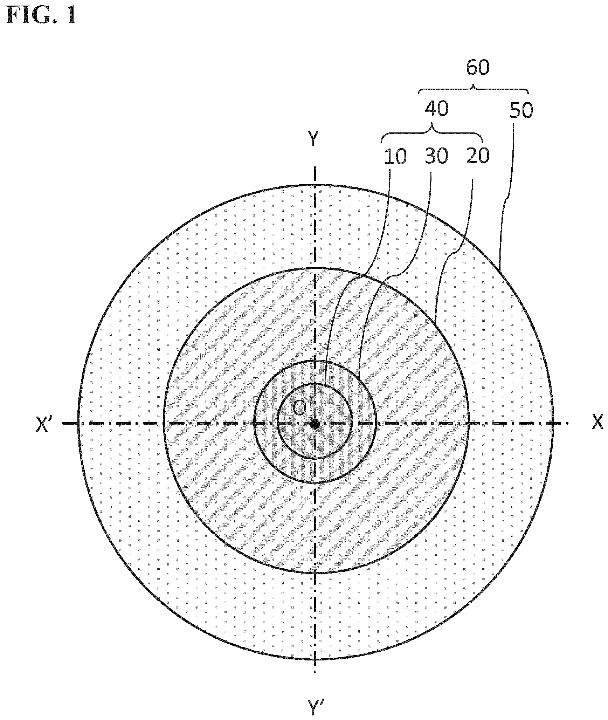

FIG. 1 is a schematic diagram illustrating a conventional multifocal lens in plan view.

In FIG. 1, the lens is viewed from above in an optical axis direction in a state where the lens is placed on a horizontal table with a front surface (a convex surface) of the lens facing upward. This similarly applies hereinafter with respect to plan views. A distance between points on the lens in the plan view will be referred to as a "plan view distance". The reference numeral 10 denotes the near-vision portion, the reference numeral 20 denotes the far-vision portion, the reference numeral 30 denotes the intermediate portion, the reference numeral 40 denotes an optical portion, the reference numeral 50 denotes a peripheral portion, and the reference numeral 60 denotes the multifocal lens. The reference numeral with the last digit 0 deleted corresponds to the respective part of the lens of the present embodiment which will be described below. The reference numerals will be omitted hereinafter.

As illustrated in FIG. 1, the near-vision portion is arranged at the center, the annular intermediate portion is arranged along an outer edge of the near-vision portion, and the far-vision portion is arranged along an outer edge of the intermediate portion such that the near-vision portion, the intermediate portion, and the far-vision portion have a common center that is an optical center O of the lens. In this example, the optical center O is made to match a geometric center. Thus, the optical portion that includes the near-vision portion, the intermediate portion, and the far-vision portion is configured. Further, there is an annular peripheral portion at the further outer edge of the optical portion. The peripheral portion usually has a flange shape that can easily enter the back side of an eyelid when the lens is placed on a cornea. That is, the lens of this example is configured by the optical portion and the peripheral portion. However, the optical portion and the peripheral portion are each distinguished to perform the above-mentioned function, and there is not a clear visible boundary such as a step between the optical portion and the peripheral portion.

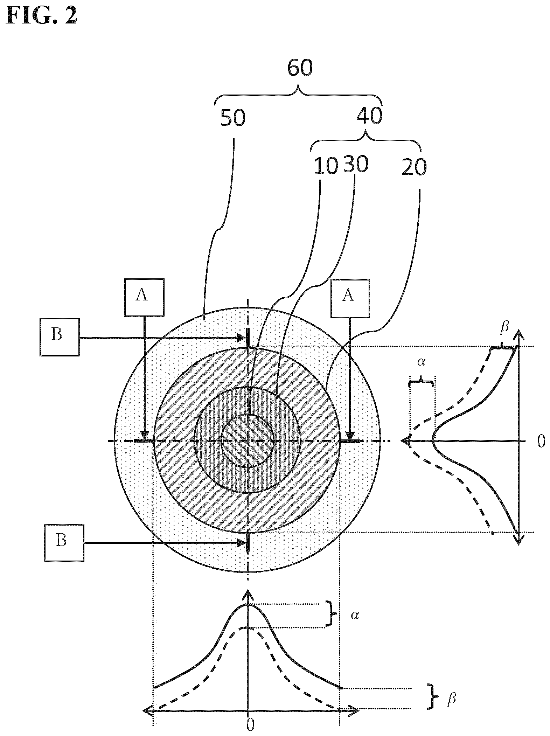

FIG. 2 is a schematic diagram of a toric multifocal lens as a reference example in plan view: a schematic view of a power distribution (tangential) in the X-direction cross-section (bottom panel); and a schematic view of a power distribution (tangential) in the Y-direction cross-section (right panel), for the lens. In both power distributions, the vertical axis represents power (unit: diopter (D)), and horizontal axis represents a distance (mm) from the optical center. In the figure (right panel) illustrating the power distribution in the Y-direction cross-section, the up-down axis corresponds to the horizontal axis, and the left-right axis corresponds to the vertical axis. In both power distributions, the solid line indicates the power in the horizontal (left-right) direction and the dashed line indicates the power in the vertical (up-down) direction.

In FIG. 2, α represents a cylinder power of the near-vision portion, and β represents a cylinder power of the far-vision portion.

The following describes a problem to be solved by the present invention.

FIG. 3 is an explanatory diagram illustrating how astigmatism occurs in the outer optical portion of the multifocal lens.

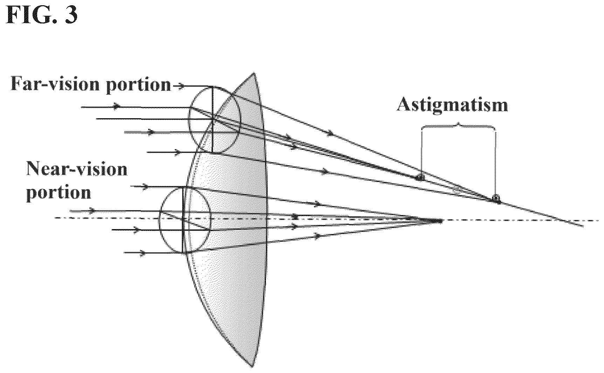

As illustrated in FIG. 3, when the power distribution of the multifocal lens is designed, the outer optical portion already includes a cylinder power. Therefore, in a toric multifocal lens of FIG. 2, the cylinder power of the outer optical portion (here, the far-vision portion) does not match the astigmatism correction power set in the far-vision portion. That is, in the toric multifocal lens of FIG. 2, it is difficult to control the cylinder power of the outer optical portion.

An object of the present invention is to enable control of the cylinder power of the outer optical portion.

### Means for solving the problem

A first aspect of the present invention provides
an ophthalmic lens including:
an optical portion that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens further including a front surface that is arranged on an object side and a rear surface that is opposed to the front surface,
wherein the front surface is a composite surface including a progressive surface and a toric surface, and
a cylinder power of the near-vision portion is equal to an astigmatism correction power set in the near-vision portion, and a cylinder power of the far-vision portion is equal to an astigmatism correction power set in the far-vision portion.

In a second aspect of the present invention that is an aspect of the first aspect,
the intermediate portion includes a portion A1 in which a power is strengthened and thereafter weakened when the lens is viewed in an X direction from the center toward the periphery, and the intermediate portion includes a portion A1' in which the power is strengthened and thereafter weakened when the lens is viewed in an X' direction from the center toward the periphery, the X' direction being exactly opposite to the X direction, the power being strengthened in the portion A1 and the portion A1' to be stronger than the far-vision power of the far-vision portion that is arranged in the ring shape along the outer edge of the intermediate portion or the near-vision power of the near-vision portion that is arranged in the ring shape along the outer edge of the intermediate portion.

In a third aspect of the present invention that is an aspect of the second aspect,
the near-vision portion or the far-vision portion that is arranged at the center of the optical portion includes a portion A2 in which a power is strengthened and thereafter weakened when the lens is viewed in the X direction from the center toward the periphery, and the near-vision portion or the far-vision portion that is arranged at the center of the optical portion includes a portion A2' in which the power is strengthened and thereafter weakened when the lens is viewed in the X' direction from the center toward the periphery, the X' direction being exactly opposite to the X direction.

In a fourth aspect of the present invention that is an aspect of the second or third aspect,
a cylinder power of the near-vision portion is equal to a cylinder power of the far-vision portion.

In a fifth aspect of the present invention that is an aspect of any one of the first to fourth aspects,
the ophthalmic lens is a contact lens.

In a sixth aspect of the present invention that is an aspect of any one of the first to fourth aspects,
the ophthalmic lens is an intraocular lens.

A seventh aspect of the present invention provides
a method of designing an ophthalmic lens including an optical portion that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens further including a front surface that is arranged on an object side and a rear surface that is opposed to the front surface,
wherein the front surface is a composite surface including a progressive surface and a toric surface, and
a cylinder power of the near-vision portion is made equal to a cylinder power of the far-vision portion.

In an eighth aspect of the present invention that is an aspect of the seventh aspect,
the intermediate portion includes a portion A1 in which a power is strengthened and thereafter weakened when the lens is viewed in an X direction from the center toward the periphery, and the intermediate portion includes a portion A1' in which the power is strengthened and thereafter weakened when the lens is viewed in an X' direction from the center toward the periphery, the X' direction being exactly opposite to the X direction, the power being strengthened in the portion A1 and the portion A1' to be stronger than the far-vision power of the far-vision portion that is arranged in the ring shape along the outer edge of the intermediate portion or the near-vision power of the near-vision portion that is arranged in the ring shape along the outer edge of the intermediate portion.

In a ninth aspect of the present invention that is an aspect of the eighth aspect,
the near-vision portion or the far-vision portion that is arranged at the center of the optical portion includes a portion A2 in which a power is strengthened and thereafter weakened when the lens is viewed in the X direction from the center toward the periphery, and the near-vision portion or the far-vision portion that is arranged at the center of the optical portion includes a portion A2' in which the power is strengthened and thereafter weakened when the lens is viewed in the X' direction from the center toward the periphery, the X' direction being exactly opposite to the X direction.

In a tenth aspect of the present invention that is an aspect of the eighth or ninth aspect,
a cylinder power of the near-vision portion is equal to a cylinder power of the far-vision portion.

In an eleventh aspect of the present invention that is an aspect of any one of the seventh to tenth aspects,
the ophthalmic lens is a contact lens.

In a twelfth aspect of the present invention that is an aspect of any one of the seventh to tenth aspects,
the ophthalmic lens is an intraocular lens.

A 13^{th} aspect of the present invention provides
a method of manufacturing an ophthalmic lens, including:
a design step of designing an ophthalmic lens using the method of designing an ophthalmic lens according to any one of the seventh to twelfth aspects of the present invention; and
a processing step of manufacturing the designed ophthalmic lens using a processing device.

A 14^{th} aspect of the present invention provides
an ophthalmic lens set including a plurality of ophthalmic lenses that each include an optical portion that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens further including a front surface that is arranged on an object side and a rear surface that is opposed to the front surface,
wherein the front surface is a composite surface including a progressive surface and a toric surface, and
a cylinder power of the near-vision portion is equal to a cylinder power of the far-vision portion.

### Advantage of the invention

The present invention enables control of the cylinder power of the outer optical portion.

### Brief description of the drawings

FIG. 1 is a schematic diagram illustrating a conventional multifocal lens in plan view.
FIG. 2 is a schematic diagram of a toric multifocal lens as a reference example in plan view: a schematic view of a power distribution (tangential) in the X-direction cross-section (bottom panel); and a schematic view of a power distribution (tangential) in the Y-direction cross-section (right panel), for the lens.
FIG. 3 is an explanatory diagram illustrating how astigmatism occurs in the outer optical portion of the multifocal lens.

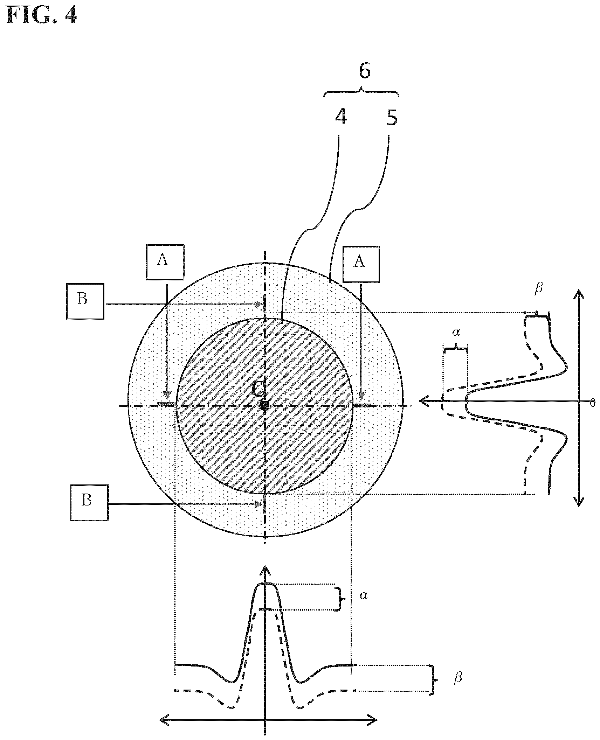

FIG. 4 is a schematic diagram of an ophthalmic lens of Example 1 in plan view: a schematic view of a power distribution in the X-direction cross-section (bottom panel); and a schematic view of a power distribution in the Y-direction cross-section (right panel), for the lens.

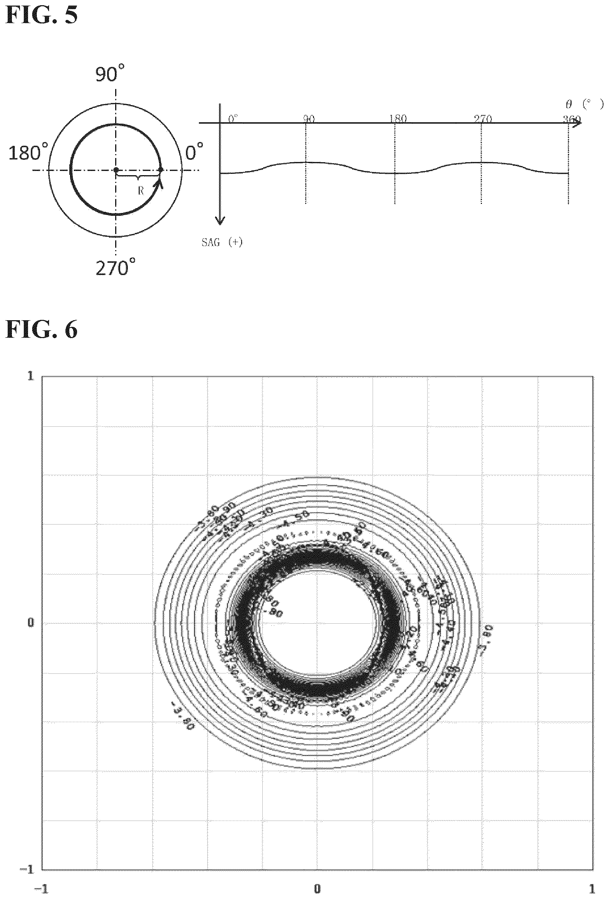

FIG. 5 is a schematic diagram illustrating the surface height (sag value) in a circumferential direction around the optical center O for the ophthalmic lens of Example 1, where the horizontal axis indicates the rotation angle (unit: °) and the vertical axis (downward) indicates the sag value.
FIG. 6 is a diagram illustrating a power distribution of the ophthalmic lens of Example 1 in plan view.

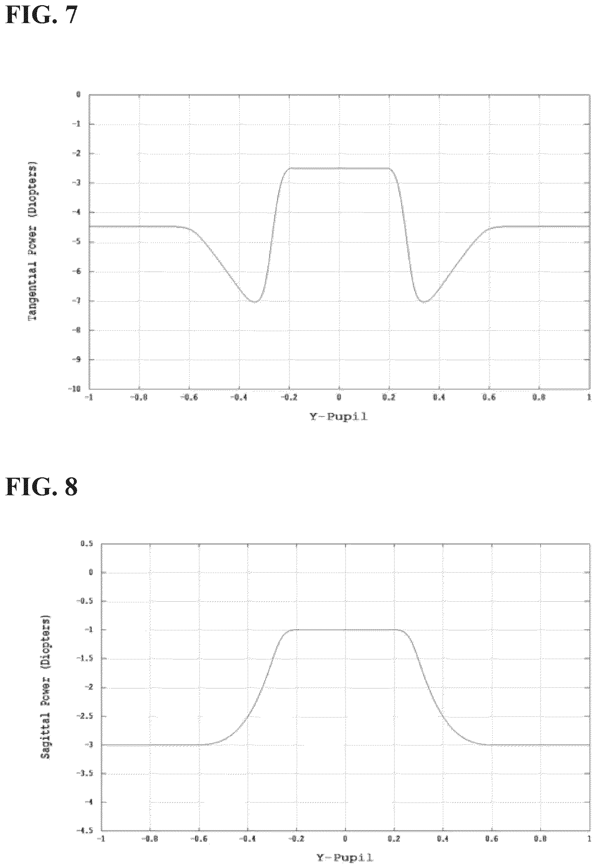

FIG. 7 illustrates a tangential power distribution in the Y-direction cross-section, for the ophthalmic lens of Example 1.
FIG. 8 illustrates a sagittal power distribution in the Y-direction cross-section, for the ophthalmic lens of Example 1.

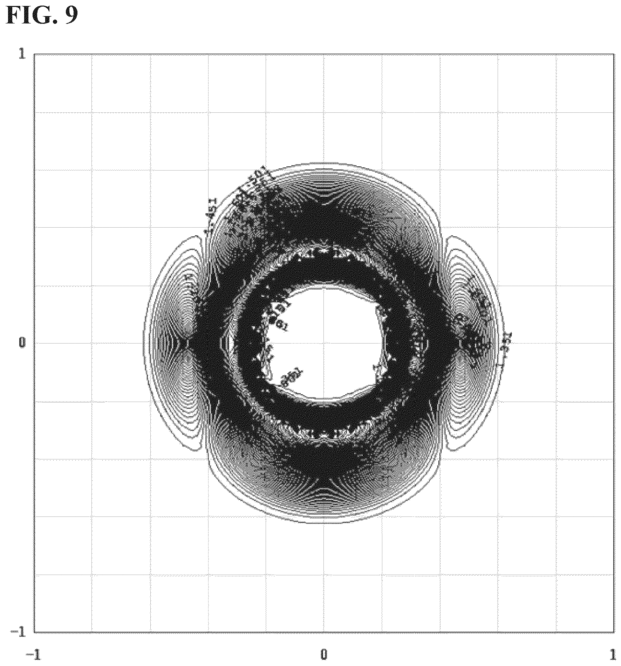

FIG. 9 is a diagram illustrating a cylinder power distribution of the ophthalmic lens of Example 1 in plan view.

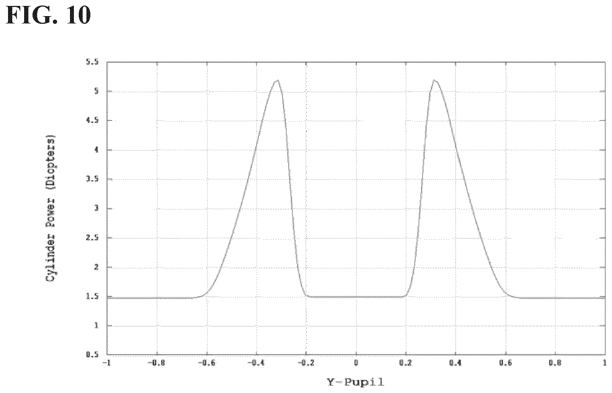

FIG. 10 illustrates a cylinder power distribution in the Y-direction cross-section, for the ophthalmic lens of Example 1.

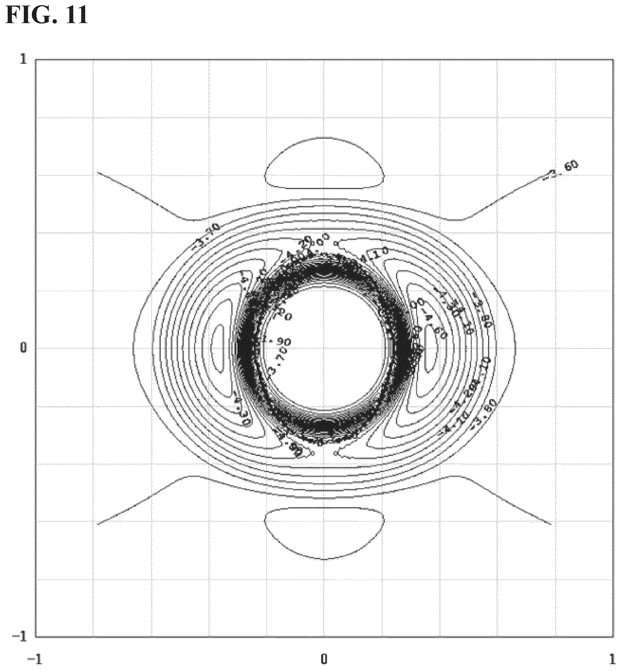

FIG. 11 is a diagram illustrating a power distribution of the ophthalmic lens of Example 2 in plan view.

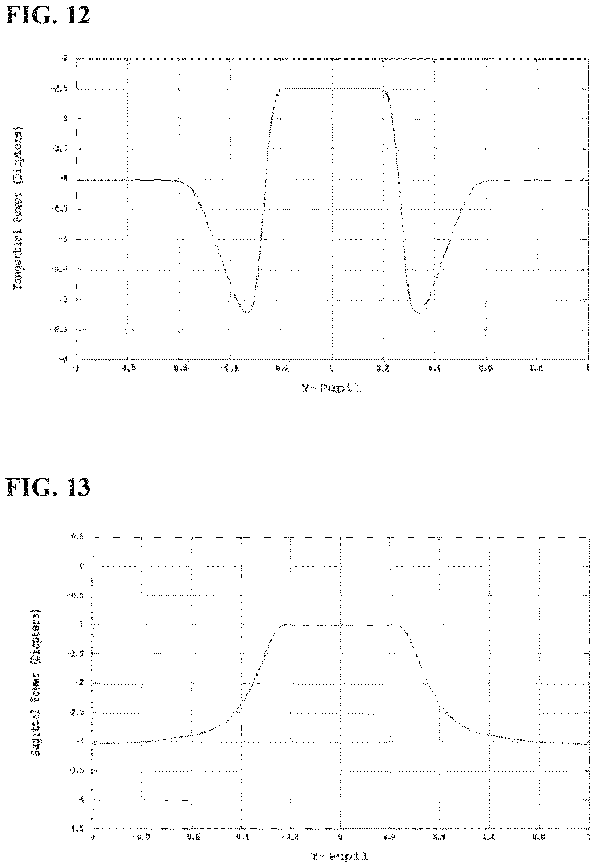

FIG. 12 illustrates a tangential power distribution in the Y-direction cross-section, for the ophthalmic lens of Example 2.
FIG. 13 illustrates a sagittal power distribution in the Y-direction cross-section, for the ophthalmic lens of Example 2.

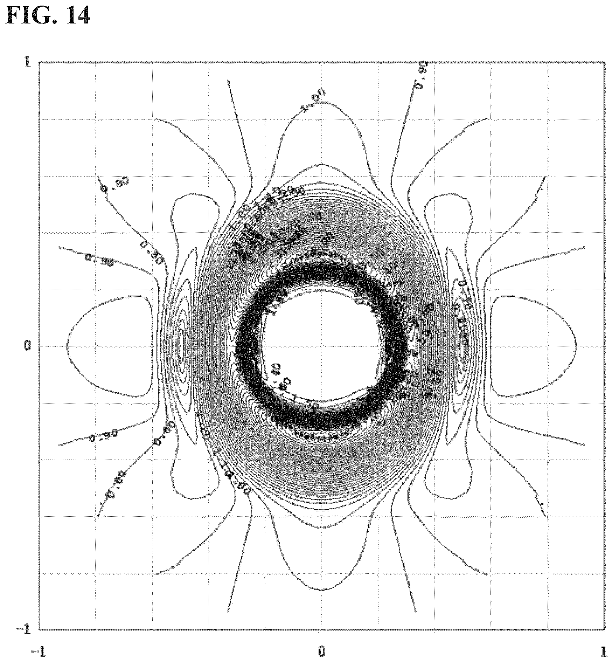

FIG. 14 is a diagram illustrating a cylinder power distribution of the ophthalmic lens of Example 2 in plan view.

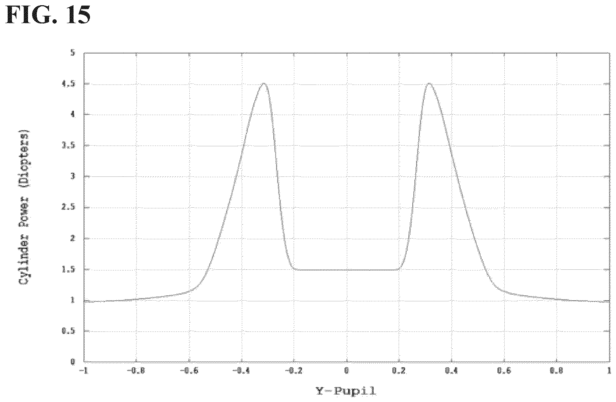

FIG. 15 illustrates a cylinder power distribution in the Y-direction cross-section, for the ophthalmic lens of Example 2.

### Detailed description of the invention

Embodiments of the present invention will be described in detail hereafter, with reference to the drawings.

The present embodiment will be described in the order shown below.
1. Contact lens
   1-1. Multifocal contact lens (multifocal lens)
   1-2. Preferred example 1
   1-3. Preferred example 2
   1-4. Preferred example 3
   1-5. Other contact lenses
2. Design method (manufacturing method) of contact lens
3. Intraocular lens (IOL) and design method (manufacturing method) thereof
4. Ophthalmic lens set
5. Modified example

Regarding a configuration which is not described below, a known configuration may be adopted suitably. Further, in the present specification, "to" refers to a predetermined value or more and a predetermined value or less.

In addition, the ophthalmic lens mentioned here (lens body of a contact lens or an intraocular lens) has two surfaces facing each other. When a wearer wears the ophthalmic lens, one located on the retina side is referred to as "a rear surface", and one located on the opposite object side is referred to as "a front surface".

Also, in the present specification, the term "power" refers to an optical power (unit: diopter [D]).

Unless otherwise specified, descriptions regarding shapes in the present specification refers to the shape of each region (near-vision portion, far-vision portion, intermediate portion) in plan view. Plan view is also a frontal view of a wearer wearing the ophthalmic lens, where the direction towards the top and bottom is also called as up and down directions, respectively, and the horizontal direction is also called as left-right directions. The left and right directions are respectively defined as the directions when the wearer is viewed from the front. However, when the ophthalmic lens is a contact lens, it is not unusual for the contact lens to rotate on the cornea. The present invention is not limited to strictly up-down or left-right directions.

In this specification, the optical center O is referred to as the inner side and the outer edge side of the ophthalmic lens is referred to as the outer side. The direction from the center toward the periphery is a radial direction from the optical center O (which matches the geometric center, for example), and hereinafter also referred to simply as a "radial direction". A direction concentric with the optical center O and perpendicular to the radial direction is also referred to as a "circumferential direction".

A direction exactly opposite to the X direction, from the center toward the periphery of the ophthalmic lens, is defined as the X' direction. As an example, the X direction is defined as the right direction, and the X' direction is defined as the left direction.

A direction perpendicular to the X direction is defined as the Y direction.

A direction exactly opposite to the Y direction, from the center toward the periphery of the ophthalmic lens, is defined as the Y' direction. As an example, the Y direction is defined as the upward direction, and the Y' direction is defined as the downward direction.

The rotation angle from the optical center O is a counterclockwise angle starting from the X direction, with the X direction being 0°, the Y direction being 90°, the X' direction being 180°, and the Y' direction being 270°.

### <1. Contact lens>

### 1-1. Multifocal contact lens (multifocal lens)

In the present embodiment, a multifocal contact lens (a multifocal lens, hereinafter also simply referred to as a "lens") will be mainly described as an example.

The lens in the present embodiment includes: a substantially circular optical portion mainly contributing to optical performance; and an annular peripheral portion disposed at the peripheral edge of the optical portion, similarly to the conventional lens described above.

As described above, the peripheral portion usually has a flange shape that is easy to enter the back of an eyelid when the lens is placed on the cornea.

The optical portion includes a near-vision portion that has a near-vision power for seeing a near distance, a far-vision portion that has a far-vision power for seeing a distance (including infinity) that is farther than the near distance, and an annular intermediate portion that connects the near-vision portion and the far-vision portion to each other. Note that the intermediate portion is a region in which the power continuously changes, the curvature in the radial direction and the curvature in the circumferential direction continuously change, and there is no step in a lens surface. The intermediate portion suppresses the occurrence of multiple images.

Note that there is no particular limitation on the near distance referred to in the present specification so long as the near distance is nearer than a far distance.

Of course, the near distance may also be an absolute distance (e.g., 100 cm or less, or 40 cm, which is a reading distance, or less) that is near.

In an example described in the present embodiment, the near-vision portion is arranged at the center, the annular intermediate portion is arranged along an outer edge of the near-vision portion, and the far-vision portion is arranged in a ring shape along an outer edge of the intermediate portion. That is, in this example, the "central optical portion" is the near-vision portion, and the "outer optical portion" is the far-vision portion.

This example shows an example of making the optical center O match the geometric center of a lens. However, the present invention is not limited thereto (the same applies hereafter).

The near-vision portion in the optical portion of the lens according to the present embodiment is a portion between a point at which the power lastly decreases from the near-vision power when the lens is viewed from the optical center O in an X direction (toward the outer periphery) and a point at which the power lastly decreases from the near-vision power when the lens is viewed from the optical center O in an X' direction. The expression "lastly" is used in view of a case where a portion may partially exist in which the power is smaller than the near-vision power at the center of the near-vision portion.

When the lens according to the present embodiment is viewed in the X direction, the annular intermediate portion in the optical portion of the lens refers to a portion from the outer edge of the near-vision portion to a point at which the power decreases to the far-vision power. Note that it refers to a portion from the outer edge of the near-vision portion to a point at which the power decreases, then further decreases to be smaller than the far-vision power, and thereafter increases to the far-vision power, in the preferred example 2 described below. In either case, the same is true when the lens is viewed in the X' direction.

The annular far-vision portion in the optical portion of the lens according to the present embodiment is a portion other than the near-vision portion and the intermediate portion, and when the lens is viewed in the X direction and the X' direction, the far-vision portion is a portion that follows the intermediate portion in which the power decreases to the far-vision power.

As described above, in the lens according to the present embodiment, the near-vision portion is arranged at the center, the annular intermediate portion is arranged along the outer edge of the near-vision portion, and the far-vision portion is arranged in the ring shape along the outer edge of the intermediate portion. Accordingly, the power at the optical center O is set to be higher than the power in the far-vision portion. In the present specification, "the power being higher" means the value of the power being larger. This similarly applies hereinafter with respect to "high" and "low" regarding the power.

As a prescription of the lens, values of the far-vision power S and the additional power ADD (and the astigmatic power C in cases where astigmatism is corrected) are usually given, and the near-vision power is a value (S + ADD) (the unit of each power is [D], this similarly applies hereinafter). In the near-vision portion, a power in the vicinity of the optical center O is taken to be the value of the near-vision power. Note that it is also possible to set the power at the position of the optical center O to the value of the near-vision power (i.e., power at the optical center O = near-vision power), and when the optical center O is shifted from the geometric center, the power at the geometric center may slightly differ from the value of the near-vision power.

In the present embodiment, the front surface is a composite surface including a progressive surface and a toric surface, the cylinder power of the near-vision portion is equal to the astigmatism correction power which is set in the near-vision portion, and the cylinder power of the far-vision portion is equal to the astigmatism correction power which is set in the far-vision portion.

The "cylinder power of the near-vision portion" refers to the astigmatism correction power in the near-vision portion. More specifically, the "cylinder power of the near-vision portion" refers to an absolute value of the difference between the cylinder power in the radial direction (tangential) in the near-vision portion and the cylinder power in the direction perpendicular to the radial direction (circumferential direction, sagittal).

Similarly, the "cylinder power of the far-vision portion" refers to an absolute value of the difference between the cylinder power in the radial direction (tangential) in the far-vision portion and the cylinder power in the direction perpendicular to the radial direction (circumferential direction, sagittal).

In the present embodiment, the cylinder power of the near-vision portion is equal to the astigmatism correction power set in the near-vision portion. Similarly, the cylinder power of the far-vision portion is equal to the astigmatism correction power which is set in the far-vision portion. The term "equal" includes both perfect agreement and within a tolerance (± 0.12 D). In most cases, the astigmatism correction power set in the near-vision portion and the astigmatism correction power set in the far-vision portion may be uniformly the same (specifically, the astigmatism correction power for the far vision is used), or they may be different. The cylinder power of the near-vision portion and the cylinder power of the far-vision portion may be equal to or different from each other. When the astigmatism correction power set in the near-vision portion and the astigmatism correction power set in the far-vision portion are different from each other, the cylinder power of the near-vision portion and the cylinder power of the far-vision portion will spontaneously differ from each other.

Incidentally, the prescription values of the wearer's information are listed in the specifications of the ophthalmic lenses. In other words, with the specifications, the ophthalmic lens can be identified as an ophthalmic lens based on the prescription value of the wearer's information. Ophthalmic lenses are usually supplied as a set with specifications. Therefore, the technical concept of the present invention is also reflected in the progressive power lenses that are supplied with specifications, and the same is true for the set of ophthalmic lenses and specifications.

As described above, unintended cylinder power occurs on the peripheral side of the optical portion having a progressive structure. In order to eliminate such an unintended cylinder power, a toric surface that cancels the unintended cylinder power is synthesized with respect to the progressive structure. In the present embodiment, the progressive structure is realized by the front surface. Then, the toric surface is synthesized with respect to the front surface which is a progressive surface. For the synthesis of progressive and toric surfaces, the entire description of WO 97/019382 (Japanese patent No. 3852116) can be referred to. When the progressive structure is realized by the front surface, the shape of the rear surface is not limited, but as an example, the rear surface (at least the rear surface side of the optical portion) is a spherical surface or a toric surface.

The present embodiment suppresses the generation of unintended cylinder power in the outer optical portion. The specific shape of the front surface is not particularly limited, as long as the shape cancels the unintended cylinder power generated in the outer optical portion. Preferred examples of the specific aspect will be described below.

### 1-2. Preferred example 1

The present embodiment may apply the invention described in WO 2020/0753 12 (Japanese Patent No. 6559866) as a preferred example.

By employing the preferred example 1, it is possible to secure a wide region in which the cylinder power is small in the far-vision portion or the near-vision portion that is arranged along the outer edge of the intermediate portion. In the present embodiment, this effect is utilized to enable control of the cylinder power of the outer optical portion.

As described in the problems to be solved by the present invention, when the power distribution of the toric multifocal lens of FIG. 2 is designed, the outer optical portion already includes a cylinder power. Therefore, in a toric multifocal lens of FIG. 2, the cylinder power of the outer optical portion (here, the far-vision portion) does not match the astigmatism correction power set in the far-vision portion.

Therefore, by employing the preferred example 1, it becomes possible to control the cylinder power already included in the outer optical portion.

Originally, the preferred example 1 has technical characteristics in that the unintended cylinder power is offset in the far-vision portion or the near-vision portion along the outer edge of the intermediate portion, consequently securing a wide region in which the cylinder power is small. One of the characteristics of the present embodiment resides in that this technical characteristics are utilized to control the cylinder power (e.g., to offset the cylinder power) that no longer matches the astigmatism correction power, and consequently, a cylinder power of the near-vision portion is made equal to an astigmatism correction power set in the near-vision portion, and a cylinder power of the far-vision portion is made equal to an astigmatism correction power set in the far-vision portion.

Hereinafter, a summary of the contents of the International Publication is provided, the entire contents of which can be incorporated herein. Hereinafter, in order to simplify the description of the present specification, the case where the far-vision portion is arranged at the center and the near-vision portion is arranged in a ring shape along the outer edge of the center is omitted.

Preferred examples of the present embodiment will be described below.

"The intermediate portion includes a portion A1 in which a power is strengthened and thereafter weakened when the lens is viewed in the X direction, and the intermediate portion includes a portion A1' in which the power is strengthened and thereafter weakened when the lens is viewed in an X' direction from the optical center O toward the periphery, the X' direction being exactly opposite to the X direction, the power being strengthened in the portion A1 and the portion A1' to be stronger than the far-vision power of the far-vision portion that is arranged in a ring shape along the outer edge of the intermediate portion or the near-vision power of the near-vision portion that is arranged in a ring shape along the outer edge of the intermediate portion,
the intermediate portion includes a portion B 1 in which a power is strengthened and thereafter weakened when the lens is viewed in a Y direction, and the intermediate portion includes a portion B1' in which the power is strengthened and thereafter weakened when the lens is viewed in a Y' direction from the optical center O toward the periphery, the Y' direction being exactly opposite to the Y direction, the power being strengthened in the portion B1 and the portion B1' to be stronger than the far-vision power of the far-vision portion that is arranged in the ring shape along the outer edge of the intermediate portion or the near-vision power of the near-vision portion that is arranged in the ring shape along the outer edge of the intermediate portion,
the portion A of the optical portion is also the portion A1 and the portion A1', and
the portion B of the optical portion is also the portion B1 and the portion B 1'."

In the present specification, in cases where a near-vision portion is arranged at the center and a far-vision portion is arranged in a ring shape along the outer edge of the center, "strengthening a far-vision power" in the far-vision portion means strengthening the power in a direction that makes it possible to see a farther distance, i.e., in the minus direction, and means reducing the power (e.g., 0.00 D → - 0.10 D). To the contrary, "weakening the far-vision power" means weakening the power in a direction that makes it difficult to see a far distance, i.e., in the plus direction, and means increasing the power (e.g., - 0.10 D → 0.00 D).

On the other hand, in cases where a far-vision portion is arranged at the center and a near-vision portion is arranged in a ring shape along the outer edge of the center, "strengthening a near-vision power" in the near-vision portion means strengthening the power in a direction that makes it possible to see a nearer distance, i.e., in the plus direction, and means increasing the power (e.g., 5.00 D → 5.10 D). To the contrary, "weakening the near-vision power" means weakening the power in a direction that makes it difficult to see a near distance, i.e., in the plus direction, and means reducing the power (e.g., 5.10 D → 5.00 D).

That is, in a case where which of the far-vision portion and the near-vision portion is to be arranged along the outer edge of the center is not determined yet, "strengthening the power" means strengthening the far-vision power or the near-vision power, and "weakening the power" means weakening the far-vision power or the near-vision power.

The intermediate portion of the lens according to the present embodiment has a shape with which the power is strengthened for far vision to be stronger than the far-vision power and thereafter weakened to reach the far-vision power when a portion A1 is viewed in the X direction and a portion A1' is viewed in the X' direction. The portions A1 and A1' referred to here are portions in which, after the power has decreased, the power (preferably, monotonously) decreases to be smaller than the far-vision power and thereafter again (preferably, monotonously) increases up to the far-vision power in the intermediate portion when the portion A1 is viewed in the X direction, for example.

In the lens according to the present embodiment, it is preferable that the cylinder power (unit: diopter) satisfies the following conditions when the lens is viewed in the X direction and the X' direction.

### <Condition 1-1>

(cylinder power at a point at which the power reaches the far-vision power as a result of being weakened after being strengthened for far vision to be stronger than the far-vision power in the portion A1) ≤ 0.30 D (preferably 0.25 D, more preferably 0.20 D, and further preferably 0.15 D)

### <Condition 2-1>

(cylinder power at a point at which the power reaches the far-vision power as a result of being weakened after being strengthened for far vision to be stronger than the far-vision power in the portion A1') ≤ 0.30 D (preferably 0.25 D, more preferably 0.20 D, and further preferably 0.15 D)

If these conditions are satisfied, it is possible to reliably secure a wide region in which the cylinder power is small as an absolute value in the far-vision portion that is arranged along the outer edge of the intermediate portion.

The conditions 1-1 and 2-1 may also be replaced with the following conditions 1'-1 and 2'-1 or the following conditions 1'-1 and 2'-1 may also be added to the conditions 1-1 and 2-1.

### <Condition 1'-1>

(cylinder power at a point at which the power reaches the far-vision power as a result of being weakened after being strengthened for far vision to be stronger than the far-vision power in the portion A1) / (maximum cylinder power in the intermediate portion) ≤ 0.30 (preferably ≤ 0.25, more preferably ≤ 0.20, and further preferably ≤ 0.15)

### <Condition 2'-1>

(cylinder power at a point at which the power reaches the far-vision power as a result of being weakened after being strengthened for far vision to be stronger than the far-vision power in the portion A1') / (maximum cylinder power in the intermediate portion) ≤ 0.30 (preferably ≤ 0.25, more preferably ≤ 0.20, and further preferably ≤ 0.15)

If these conditions are satisfied, it can be ensured that the cylinder power generated in the intermediate portion immediately decreases when the lens is viewed from the center toward the periphery of the lens (i.e., in the X direction and the X' direction).

Note that it is preferable that the power has a local minimum value only at a point in the portion A1 and has a local minimum value only at a point in the portion A1'. With this provision, there is no need to provide many small recessed portions when viewed in the power plot and a lens design can be kept from becoming complex. However, this provision is not essential, and a configuration is also possible in which the power has local minimum values at two or three points, for example.

Also, it is preferable that a plan view distance L between a point (outermost point) at which the power has a local minimum value in the portion A1 and a point (outermost point) at which the power has a local minimum value in the portion A1' is 2.0 to 5.0 mm. The lower limit is more preferably 2.2 mm, and the upper limit is more preferably 4.8 mm. With this provision, it is possible to reliably set positions from which the decreased power starts to increase to appropriate positions, and to provide the far-vision portion having a small cylinder power based on specific dimensions. However, the numerical value range described above does not necessarily have to be satisfied, and the plan view distance L may be appropriately set according to the type of the lens.

Also, it is preferable that a ratio of a difference between each local minimum power value and the far-vision power to a difference between the near-vision power and the far-vision power in the portion A1 and the portion A1' is 0.15 or more and 1.0 or less. The lower limit of the ratio is more preferably 0.25, further preferably 0.30, and particularly preferably 0.40, and the upper limit of the ratio is more preferably 0.90, further preferably 0.80, and particularly preferably 0.70.

For example, when the lens is viewed in the X direction, the lens may also have a power plot in which the power monotonously increases or decreases, rather than becoming constant, after the power has changed in the intermediate portion as described above and reached the far-vision power. However, the far-vision portion is the portion having the far-vision power for seeing a predetermined distance that is farther than the near distance, and accordingly, an excessive change in the power is not preferable, and it is preferable that a change in the power in the far-vision portion is within a range of ± 0.50 D (preferably ± 0.25 D) with respect to the far-vision power.

Similarly, in the near-vision portion arranged at the center, the lens may also have a power plot in which the power increases or decreases from the near-vision power. However, the near-vision portion is the portion having the near-vision power for seeing the near distance, and accordingly, an excessive change in the power is not preferable, and it is preferable that a change in the power in the near-vision portion is within a range of ± 0.50 D (preferably ± 0.25 D) with respect to the near-vision power. Furthermore, if the power becomes smaller than the near-vision power in the near-vision portion, a sufficient power for seeing the near distance cannot be obtained, which is not preferable. Therefore, it is further preferable that a change in the power in the near-vision portion is within a range of + 0.50 D (preferably + 0.25 D) with respect to the near-vision power. Also, when the lens is viewed from the optical center O in the X direction and the X' direction, the near-vision portion may also have a shape with which the power is strengthened for near vision to be stronger than the near-vision power in order to sufficiently achieve the near-vision power in the near-vision portion.

In the preferred example 2, the cylinder power is 0.25 D or less, mostly close to 0, in the far-vision portion or the near-vision portion along the outer edge of the intermediate portion. That is, the cylinder power generated in the intermediate portion immediately decreases and a wide region in which the cylinder power is small is secured in the far-vision portion or the near-vision portion that is arranged along the outer edge of the intermediate portion.

Note that, in the lens according to the present embodiment, it is possible to make the cylinder power low in a wide area of the far-vision portion owing to the configurations described above. Specifically, when the straight line X-X' is rotated relative to the lens about the optical center O from 0° to 180°, a portion of the far-vision portion in which the cylinder power is 0.50 D or less is preferably 80 area% or more, more preferably 90 area% or more, and further preferably 95 area% or more.

In the present specification, "area%" means a percentage of a total area of the portions having the above shape when a straight line X-X' is rotated relative to the lens about the optical center O from 0 to 180° (e.g., two fan-shaped portions surrounded by the optical center O and the arc of the outermost edge of the optical portion (portion A1 at 0° to 180°, portion A1' at 180° to 360°)) in plan view when viewed from the optical center O, with respect to the area of the optical portion similarly in plan view.

Incidentally, there is no boundary that can be visually recognized between the optical portion and the peripheral portion in the lens as described above, but the optical portion and the peripheral portion can be distinguished using a device (power meter) for measuring the power of the lens.

### 1-3. Preferred example 2

The present embodiment may apply the invention described in WO 2018/138931 (Japanese Patent No. 6188974) as a preferred example. Hereinafter, a summary of the contents of the International Publication is provided, the entire contents of which can be incorporated herein. Hereinafter, in order to simplify the description of the present specification, the case where the far-vision portion is arranged at the center and the near-vision portion is arranged in a ring shape along the outer edge of the center is omitted.

Preferred examples of the present embodiment will be described below.

"The central optical portion includes a portion A2 in which a power is strengthened and thereafter weakened when the lens is viewed in the X direction, and the central optical portion includes a portion A2' in which the power is strengthened and thereafter weakened when the lens is viewed in an X' direction from the optical center O toward the periphery, the X' direction being exactly opposite to the X direction,
the central optical portion includes a portion B2 in which a power is strengthened and thereafter weakened when the lens is viewed in a Y direction, and the central optical portion includes a portion B2' in which the power is strengthened and thereafter weakened when the lens is viewed in a Y' direction from the optical center O toward the periphery, the Y' direction being exactly opposite to the Y direction,
the portion A of the optical portion is also the portion A2 and the portion A2', and
the portion B of the optical portion is also the portion B2 and the portion B2'."

Preferably, there is only one point where the power is locally maximum in the portion A2, and there is only one point where the power is locally maximum in the portion A2'. In other words, it is preferable that there are two upward-convex portions (i.e., one concave portion) when viewed in the power plot. With this provision, it is not necessary to provide a large number of small convex portions when viewed in the power plot.

Further, it is preferable that the plan view distance L between the point where the power is locally maximum in the portion A2 and the point where the power is locally maximum in the portion A2' is 1.0 to 2.8 mm. The lower limit is more preferably 1.2 mm, still more preferably 1.4 mm, and very preferably 1.6 mm, and the upper limit is more preferably 2.6 mm, still more preferably 2.4 mm. This provision makes it possible to ensure that the position where the power is increased and then decreased is appropriate. However, this is not essential, and the plan view distance L may be appropriately set according to the type of lens.

Preferably, the difference between the local maximum of the power and the near-vision power in the portion A2 is 0.05 to 0.25 D, and also preferably, the difference between the local maximum of the power and the near-vision power in the portion A2' is 0.05 to 0.25 D. The lower limit of them is more preferably 0.10 D, more preferably 0.12 D, particularly preferably 0.15 D, and the upper limit of them is more preferably 0.20D.

The present invention does not exclude a case of providing, in addition to the near-vision portion at the center and the far-vision portion at the peripheral edge, an annular near-vision portion at the further peripheral edge of the far-vision portion. Although it will be described in detail later, the same applies to a case of providing the far-vision portion at the center, the near-vision portion at the peripheral edge of the far-vision portion, and the annular far-vision portion at the further peripheral edge of the near-vision portion.

The lens of the present embodiment has a shape in which the power is strengthened for near vision to be stronger than the near-vision power and then weakened to the far-vision power in the portion A2 and the portion A2'. For example, the portion having that shape when a straight line X-X' is rotated relative to the lens about the optical center O from 0 to 180° is preferably 50 area% or more, more preferably 80 area% or more, and further preferably 90 area% or more of an entire optical portion (for convenience of explanation, also simply referred to as an optical portion).

By adopting this preferred example, a good balance can be kept between the near-vision portion and the far-vision portion provided at the outer edge of the near-vision portion while ensuring the near-vision power sufficiently in the near-vision portion when the near-vision portion is arranged at the center of the optical portion, and a good balance can be kept between the far-vision portion and the near-vision portion provided at the outer edge of the far-vision portion while ensuring the far-vision power sufficiently in the far-vision portion when the far-vision portion is arranged at the center of the optical portion.

### 1-4. Preferred example 3

In the present embodiment, the diameter of the incident pupil (hereinafter, also referred to as incident pupil diameter) of a person may be adopted regardless of age, sex, or other factors. In the present specification, the incident pupil diameter refers to a diameter formed on an ophthalmic lens (or more precisely, a front surface of the lens) by a light flux that enters the aperture hole (here, a pupil of a wearer) among the light fluxes that pass through the lens. In other words, the incident pupil diameter refers to the diameter of the circular region of the lens in plan view. As an example, the expected incident pupil diameter may be 4.0 to 5.0 mm, or may be 4.4 mm. Hereinafter, the expected incident pupil diameter is 4.4 mm. The expected incident pupil diameter is also the diameter of a virtual circle centered at the optical center O. The expected incident pupil diameter is sometimes simply referred to as the incident pupil diameter. The expected incident pupil diameter is defined assuming that the wearer wears the ophthalmic lens with the pupil center of the wearer matching the optical center.

The "outer edge of the intermediate portion fits within the expected incident pupil diameter" specifies that at least a portion of the far-vision portion, which is an outer optical portion, exists within the incident pupil diameter. The "outer edge of the outer optical portion exists outside the expected incident pupil diameter" specifies that the far-vision portion is large enough to exist outside the range of the incident pupil diameter.

In the optical portion of the ophthalmic lens according to the present embodiment, a ratio between the far-vision power group and the near-vision power group when the lens is viewed in the X direction (ratio X1) is different from a ratio between the far-vision power group and the near-vision power group when the lens is viewed in the Y direction (ratio Y1), within the expected incident pupil diameter. More specifically, the ratio X1 between the far-vision power group and the near-vision power group that constitute the power distribution of the portion A of the optical portion when the lens is viewed in the X direction is different from the ratio Y1 between the far-vision power group and the near-vision power group that constitute the power distribution of the portion B of the optical portion when the lens is viewed in the Y direction.

In the present specification, the power distribution of the optical portion refers to the following plot. The horizontal axis of the plot indicates a distance (unit: mm) from the optical center O in the X-direction cross-section in plan view of the lens. The vertical axis indicates the spherical power (unit: diopter [D]) of the lens. This similarly applies hereinafter with respect to diagrams obtained by plotting spherical powers. In the present specification, the "cross-section" passes through the optical center O unless otherwise specified.

The optical design analysis software (Zemax OpticStudio: created by Zemax, LLC) using ray tracing may be used to obtain the plot. An aperture diameter may be set to 8.0 mm, and the wavelength of light may be set to 550 nm. Hereinafter, in the present specification, examples in which these are adopted will be given unless otherwise specified.

In this aspect, the power distribution of the optical portion is composed of the far-vision power group (mass) and the near-vision power group. In this specification, a power equal to or greater than the far-vision power plus 1/2 of the additional power is classified as a near-vision power group, and the rest are classified as a far-vision power group. The far-vision power group is provided primarily by the far-vision portion, and the near-vision power group is provided primarily by the near-vision portion. Therefore, the expressions, "the far-vision power group belongs to the far-vision portion side" and "the near-vision power group belongs to the near-vision portion side" are used. The power group may be referred to as the power belt.

An example of the method to obtain the ratio X1 is described below. First, the power distribution of the optical portion is obtained. That is, the above-described plot, with the horizontal axis representing the distance from the optical center O and the vertical axis representing the spherical power of the lens, is obtained for a portion A within the expected incident pupil diameter. Then, in the above plot, the far-vision power plus 1/2 of the additional power is taken as a threshold value, and a ratio between an area NA and an area FA is X1, where NA is an area between plots considered to be the near-vision power group and the horizontal axis, and FA is an area between a portion considered to be the far-vision power group and the horizontal axis.

The above-described area NA is defined as an area between the horizontal line with the minimum power in the portion A and plots considered to be the near-vision power group for a case where the outer optical portion is the far-vision portion, and defined as an area between the horizontal line with the maximum power in the portion A and plots considered to be the near-vision power group for a case where the outer optical portion is the near-vision portion.

The above-described area FA is defined as an area between the horizontal line with the minimum power in the portion A and plots considered to be the far-vision power group for a case where the outer optical portion is the far-vision portion, and defined as an area between the horizontal line with the maximum power in the portion A and plots considered to be the far-vision power group for a case where the outer optical portion is the near-vision portion.

The ratio Y1 is obtained in the same manner as the ratio X1. The ratio X1 and the ratio Y1 may be expressed as NA:FA, NA/FA, or FA/NA.

The ratio X1 may be expressed as a ratio between the distance of the NA in the horizontal axis direction and the distance of the FA in the horizontal axis direction.

In the present specification, "the ratio X1 is different from the ratio Y1" means that they are different in accordance with at least one of the above definitions.

There is no limitation on the specific configuration of the optical portion that makes the ratio X1 different from the ratio Y1. Specific examples are given below.

In the present specification, "the ratio X1 is different from the ratio Y1" means that the power distribution of the portion A and the power distribution of the portion B do not have the same shape. It means that, among the power distributions, at least one of the power distribution of the outer optical portion (the far-vision portion in this example) and the power distribution of the intermediate portion does not have the same shape.

In the present specification, "the ratio X1 is different from the ratio Y1" can also be specifically expressed as follows.

"One of the following conditions is satisfied:
(Condition 1)
   within the expected incident pupil diameter, a width of the far-vision portion when viewed in the X direction is different from a width of the far-vision portion when viewed in the Y direction;
(Condition 2)
   while the width of the far-vision portion when viewed in the X direction matches the width of the far-vision portion when viewed in the Y direction, the band-like region in the ring of the intermediate portion has a wide portion and a narrow portion.
(Condition 3)
   while he width of the far-vision portion when viewed in the X direction matches the width of the far-vision portion when viewed in the Y direction, and the width of the band-like region in the ring of the intermediate portion has a constant width, behavior of the change in the power in the X direction is different from that in the Y direction at least when the intermediate portion is viewed in the radial direction".

The above-described conditions 1 to 3 correspond to the specific examples 1 to 3, respectively.

In addition, while condition 1 is satisfied, at least one of the following conditions may be satisfied: the band-shaped region in the ring of the intermediate portion has a wide portion and a narrow portion, and behavior of the change in the power in the X direction is different from that in the Y direction at least when the intermediate portion is viewed in the radial direction.

In either specific example, it is easier to secure the far-vision power group belonging to the far-vision portion side which is the outer optical portion or the near-vision power group belonging to the near-vision portion side which is the outer optical portion, while the near-vision portion or the far-vision portion, which is the central optical portion, performs the optical function and the intermediate portion suppresses the occurrence of multiple images.

A common provision in all of the specific examples is that the area of the near-vision portion in plan view may be 3.14 mm² or more, and the near-vision portion may encompass a circle with an area of 3.14 mm² or more centered at the optical center O. The area value described above is specified taking into consideration that it is preferable for the central optical portion (the near-vision portion in this example) to have a diameter of 2 mm. However, as shown in Examples below, the area may be 2.50 mm² or more, 2.75 mm² or more, or 3.00 mm² or more.

The outer edge of the intermediate portion fitting within a circular frame with a diameter of 4.5 mm, which is a common provision in all of the specific examples, can secure a wide far-vision portion that exist outside the intermediate portion, which is preferable. The outer edge of the intermediate portion may fit within a circle with a diameter of 4.0 mm. There is no limitation on the size of the inner edge of the intermediate portion, i.e., the outer edge of the near-vision portion, as long as the function of the near-vision portion can be fully performed. For example, the outer edge of the near-vision portion may be large enough to encompass a circle with a diameter of 2.5 mm (or 3.0 mm).

The upper limit of the size of the central optical portion (the near-vision portion in this example) may be appropriately changed to fit the wearer, but a moderate size is preferable from the viewpoint of securing the outer optical portion (the far-vision portion in this example). For example, the central optical portion may be large enough to be encompassed within a circle with a diameter of 2.5 mm.

In a case where the X direction is at a rotation angle with a predetermined value, the ratio X1 just has to be different from the ratio Y1. Then, if the far-vision power group has the smallest ratio at the ratio X1, the ratio of the far-vision power group will always be larger than X1 at the ratio Y1, at least compared to the case of the perfect circle in FIG. 1 (constant at the ratio X1 for each rotation angle). On the other hand, the effect of the present embodiment becomes more pronounced in the optical portion as the proportion of the regions where the ratio X1 is different from the ratio Y1 increases. The rotation angle from the optical center O may be used to indicate this proportion. Whether the ratio X1 is different from the ratio Y1 or not in the region may be varied for each range of the rotation angle. In such a case, the region is fan-shaped.

The larger the range of the rotation angle, the wider the fan-shaped area will be, and hence the wider the configuration of the present embodiment will be provided, which is preferable. For example, when the lens is viewed in the radial direction from the optical center O, it is preferable that the range of the rotation angle, which has the configuration of the present embodiment, is a total of 180° or more (50 area% or more of the entire optical portion), 210° or more (58 area% or more of the entire optical portion ), 240° or more (67 area% or more of the entire optical portion ), 270° or more (75 area% or more of the entire optical portion ), 300° or more (83 area% or more of the entire optical portion ), 330° or more (92 area% or more of the entire optical portion ), or 360° (i.e., entirety) (100 area% of the entire optical portion).

In the above description, the present embodiment is explained by the power distribution. However, it is also possible to specify the present embodiment by the shape (curvature) of the front surface instead of the power distribution. This is because a surface (rear surface) of a conventional lens that comes into contact with a cornea needs to have a shape corresponding to a surface (e.g., a spherical surface or a toric surface) that conforms to the shape of the cornea. Accordingly, the power needs to be adjusted by adjusting the shape of a surface (front surface) on the eyelid side. Actually, the power distribution referred to in the present specification is obtained by adjusting the shape of the front surface while forming the rear surface as a spherical surface.

Therefore, the characteristics of the power plot can also be expressed using the shape (curvature) of the front surface of the lens.

Incidentally, a preferred example for a case specified in terms of the power can also be applied to a case specified in terms of the radius of curvature, after converting the power to the radius of curvature as appropriate.

### 1-5. Other contact lenses

In the present embodiment, the multifocal contact lens is exemplified. However, a technical idea of the present invention can be applied to other contact lenses.

For example, also in multifocal toric contact lenses, the above-described behavior of power is not hindered due to the toric shape. This is because, for a toric contact lens, a uniform difference in curvature (difference between the curvature in the radial direction and the curvature in the circumferential direction) is provided in a surface of the lens, and there is no problem in making the curvature in the circumferential direction closer to the curvature in the radial direction in the intermediate portion as described as the findings of the present invention. Therefore, the technical idea of the present invention can also be applied to toric contact lenses.

The lens of the present embodiment including the portions described above can be applied whether it is a soft contact lens or a hard contact lens. However, the soft contact lens with little movement on the cornea is more preferable in providing sufficient optical performance and customer satisfaction with a wearer.

It is also possible to impart a nearsightedness progression suppressing effect to the lens according to the present embodiment. Such a lens will be referred to as a "nearsightedness progression suppressing lens". The nearsightedness progression suppressing effect is achieved by causing light that enters an eyeball to converge in front of a retina (on an object side as viewed from the retina).

This nearsightedness progression suppressing effect can be realized with a lens with far-vision center, for example. Specifically, a configuration is also possible in which a far-vision portion arranged at the center has a shape that reflects prescription values, and a near-vision portion that has a higher power than that of the far-vision portion (causing light to converge in front of the retina) is provided along an outer edge of an intermediate portion that is interposed between the far-vision portion and the near-vision portion.

Conversely, this nearsightedness progression suppressing effect can also be realized with a lens with near-vision center. Specifically, a configuration is also possible in which a near-vision portion arranged at the center causes light to converge in front of the retina, and a far-vision portion that has a lower power than that of the near-vision portion, i.e., has a shape that reflects prescription values is provided along an outer edge of an intermediate portion.

That is, in the case of a lens imparted with the nearsightedness progression suppressing effect, the effects of the present invention can be achieved if any of the regions that are concentrically arranged in the lens has a shape that reflects prescription values for a wearer. The far-vision portion and the near-vision portion (particularly, the near-vision portion) that are respectively arranged close to the center and close to the outer edge relative to the annular intermediate portion having the characteristics of the present embodiment do not necessarily need to have shapes that reflect prescription values for a wearer. Note that "a near-vision power that corresponds to a near distance" referred to in the present specification includes a power that causes light to converge in front of the retina as well as the "near-vision power for seeing a near distance", which is the expression used heretofore, i.e., a power that corresponds to a prescription value for a wearer. This may also be referred to as "a power that corresponds to a near distance" to avoid misinterpretation.

As a result of the above, according to each example of the present embodiment, when the lens is viewed from the center toward the periphery, it is possible to immediately reduce a cylinder power generated in the intermediate portion to make the cylinder power small in the far-vision portion or the near-vision portion that is arranged along the outer edge of the intermediate portion, and consequently, it is possible to secure a wide region in which the cylinder power is small in the far-vision portion or the near-vision portion that is arranged along the outer edge of the intermediate portion.

### <2. Design method (manufacturing method) of contact lens>

The above contents can be sufficiently applied to a method of designing or manufacturing a contact lens. For example, the method of designing a contact lens is as follows.

"A method of designing an ophthalmic lens including an optical portion that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens further including a front surface that is arranged on an object side and a rear surface that is opposed to the front surface,
wherein the front surface is a composite surface including a progressive surface and a toric surface, and
a cylinder power of the near-vision portion is made equal to a cylinder power of the far-vision portion."

Regarding a specific design method, it is sufficient to design using a known lens design method or a design device. Further, division into cases of (arranging the near-vision portion at the center and arranging the far-vision portion at the center) described in <1. Contact lens> and each preferred example can also be applied to <2. Design method (manufacturing method) of contact lens>. Therefore, the contents described above will not be described here, because it overlaps with the description in the <1. Contact lens>.

When the design is changed so that the outer edge of the intermediate portion is transformed from a perfect circle into an ellipse in plan view, an amount of decrease from the radius of the perfect circle to the length of the minor axis of the ellipse may be greater than an amount of increase from the radius of the perfect circle to the length of the major axis of the ellipse. Accordingly, the area of the far-vision portion can be surely larger compared to a perfect circle.

The manufacturing method includes a design step of designing an ophthalmic lens using the above-described method of designing an ophthalmic lens (combining preferred examples as appropriate depending on situations) and a processing step of manufacturing the designed ophthalmic lens using a processing device. Note that a known device of processing a lens can be used in a specific procedure of processing the lens. For a specific procedure, a known procedure may be applied. For example, the contents described in WO 02/048779 (Japanese Patent No. 503 1808) may be applied.

### <3. Intraocular lens (IOL) and design method (manufacturing method) thereof>

The technical idea of the present invention is fully applicable to an intraocular lens (IOL) and a design method (manufacturing method) thereof as well. There is no particular limitation on the intraocular lens, and the technical idea of the present invention is applicable to an intraocular lens of a type (in-the-bag) that is arranged inside of a lens capsule, an intraocular lens of a type (out-of-the-bag) that is arranged outside of the capsule, and an intraocular lens that is sewn, for example.

Note that, in a case where the technical idea of the present invention is applied to an intraocular lens, the intraocular lens only needs to include at least the optical portion. Although an annular peripheral portion may also be provided along the peripheral edge of the optical portion that mainly contributes to optical performance as described in <1-1. Multifocal contact lens (multifocal lens)>, an intraocular lens in this example is configured by the optical portion and a support portion that supports the optical portion in a lens capsule. In relatively many cases, an intraocular lens is configured by the optical portion described above and a support portion that extends from the optical portion. For the support portion, the shape of the support portion of the known intraocular lens may be adopted. However, for example, two support portions extending in an arm shape from the optical portion may be provided in the optical portion and a lens having such a configuration may be used as an intraocular lens.

Regarding the method of designing an intraocular lens (the method of manufacturing an intraocular lens), description of the design of the optical portion is omitted because it is the same as that described in <2. Design method (manufacturing method) of contact lens>. Regarding a specific method of designing an intraocular lens (method of manufacturing an intraocular lens), it is sufficient to perform the design using a known method (processing device) of designing an intraocular lens. Further, division into cases of (arranging the near-vision portion at the center and arranging the far-vision portion at the center) described in <1. Contact lens> and each preferred example can also be applied to <3. Intraocular lens (IOL) and design method (manufacturing method) thereof>. Therefore, the description is omitted here because it overlaps with the description in the <1. Contact lens>.

### <4. Ophthalmic lens set>

The contents described above are fully applicable to a contact lens set that includes a plurality of contact lenses described in the present embodiment and the intraocular lens set that includes a plurality of intraocular lenses described in the present embodiment. These lens sets are collectively referred to as "an ophthalmic lens set".

When selling at least a contact lens as a product, not only single contact lens is sold, but multiple contact lenses with a wide variety of powers and base curves (e.g., multiple contact lenses with the same base curve but different powers) are collectively sold frequently under one trade name.

Therefore, the technical idea of the present invention is sufficiently reflected on the ophthalmic lens set including a plurality of lenses exhibiting the behavior of power such as power of the contact lens (or intraocular lens etc.) of the present embodiment described in detail above.

From a different point of view, powers of all ophthalmic lens sets that constitute an ophthalmic lens set according to the present embodiment show the above-described behavior. This shows that, even if one ophthalmic lens showing the above behavior of power is produced in the conventional art, the composition is completely different between the ophthalmic lens thus produced by chance and the ophthalmic lens set of this embodiment.

The configuration of the ophthalmic lens set including a plurality of the above ophthalmic lenses is as follows. For the following configuration, the above-described preferred examples may be suitably combined.

"An ophthalmic lens set including a plurality of ophthalmic lenses that each include an optical portion that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens further including a front surface that is arranged on an object side and a rear surface that is opposed to the front surface,
wherein the front surface is a composite surface including a progressive surface and a toric surface, and
a cylinder power of the near-vision portion is equal to a cylinder power of the far-vision portion."

### <5. Modified example>

The present invention is not limited to the above examples, and the above examples and preferred examples may, of course, be combined as appropriate.

For example, the "central optical portion" may be the far-vision portion, and the "outer optical portion" may be the near-vision portion. In that case, if the terms "near-vision portion" and "far-vision portion" are interchanged in the above sentence, and the expression about the level of the power is changed accordingly, the present invention is applicable. For other contents, the contents explained in the present embodiment can be used.

### Examples

Examples of the present invention will be specifically described below. However, the present invention is not limited to Examples described below.

### <Example 1>

This example is a lens to which the preferred example 2 is applied.

In this example, the base curve (BC) is set to 8.50, the far-vision power (spherical power) S is set to - 5.00 D, the near-vision power (S + ADD) is set to - 3.00 D, and the astigmatic power C is set to - 1,50 D. That is, an additional power ADD is + 2.00 D. In the present specification, an "additional power" refers to a value obtained by subtracting a far-vision power from a near-vision power. The central thickness (CT) of the lens is 0.10 mm, and the refractive index (n) of the lens is 1.44. This similarly applies hereinafter unless otherwise stated.

FIG. 4 is a schematic diagram of an ophthalmic lens of Example 1 in plan view: a schematic view of a power distribution in the X-direction cross-section (bottom panel); and a schematic view of a power distribution in the Y-direction cross-section (right panel), for the lens. In both power distributions, the vertical axis represents power (D), and horizontal axis represents a distance (mm) from the optical center. In the present specification, the power distribution of the astigmatic power is referred to as the cylinder power distribution. The expression "power distribution" simply refers to the power distribution of the spherical power.

FIG. 5 is a schematic diagram illustrating the surface height (sag value) in a circumferential direction around the optical center O for the ophthalmic lens of Example 1, where the horizontal axis indicates the rotation angle (unit: °) and the vertical axis (downward) indicates the sag value.

FIG. 6 is a diagram illustrating a power distribution of the ophthalmic lens of Example 1 in plan view.

FIG. 7 illustrates a tangential power distribution in the Y-direction cross-section, for the ophthalmic lens of Example 1.

FIG. 8 illustrates a sagittal power distribution in the Y-direction cross-section, for the ophthalmic lens of Example 1.

FIG. 9 is a diagram illustrating a cylinder power distribution of the ophthalmic lens of Example 1 in plan view.

FIG. 10 illustrates a cylinder power distribution in the Y-direction cross-section, for the ophthalmic lens of Example 1.

For the scale in the power distribution in the present specification, + 1 indicates + 4.0 mm and - 1 indicates - 4.0 mm. The distance in the X direction from the center of the lens is also referred to as X-pupil, and the distance in the Y direction from the center of the lens is also referred to as Y-pupil.

### <Example 2>

FIG. 11 is a diagram illustrating a power distribution of the ophthalmic lens of Example 2 in plan view.

FIG. 12 illustrates a tangential power distribution in the Y-direction cross-section, for the ophthalmic lens of Example 2

FIG. 13 illustrates a sagittal power distribution in the Y-direction cross-section, for the ophthalmic lens of Example 2.

FIG. 14 is a diagram illustrating a cylinder power distribution of the ophthalmic lens of Example 2 in plan view.

FIG. 15 illustrates a cylinder power distribution in the Y-direction cross-section, for the ophthalmic lens of Example 2.

This example is similar to Example 1, except that S is set to - 3.00D, and the (cylinder) power distributions illustrated in FIG. 10 to 14 are used.

### Description of signs and numerals

10...Near-vision portion
20...Far-vision portion
30...Intermediate portion
4...Optical portion
5...Peripheral portion
6...Multifocal contact lens

## Claims

1. An ophthalmic lens comprising:
an optical portion that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens further including a front surface that is arranged on an object side and a rear surface that is opposed to the front surface,
wherein the front surface is a composite surface including a progressive surface and a toric surface, and
a cylinder power of the near-vision portion is equal to an astigmatism correction power set in the near-vision portion, and a cylinder power of the far-vision portion is equal to an astigmatism correction power set in the far-vision portion.

2. The ophthalmic lens according to claim 1,
wherein the intermediate portion includes a portion A1 in which a power is strengthened and thereafter weakened when the lens is viewed in an X direction from the center toward the periphery, and the intermediate portion includes a portion A1' in which the power is strengthened and thereafter weakened when the lens is viewed in an X' direction from the center toward the periphery, the X' direction being exactly opposite to the X direction, the power being strengthened in the portion A1 and the portion A1' to be stronger than the far-vision power of the far-vision portion that is arranged in the ring shape along the outer edge of the intermediate portion or the near-vision power of the near-vision portion that is arranged in the ring shape along the outer edge of the intermediate portion.

3. The ophthalmic lens according to claim 2,
wherein the near-vision portion or the far-vision portion that is arranged at the center of the optical portion includes a portion A2 in which a power is strengthened and thereafter weakened when the lens is viewed in the X direction from the center toward the periphery, and the near-vision portion or the far-vision portion that is arranged at the center of the optical portion includes a portion A2' in which the power is strengthened and thereafter weakened when the lens is viewed in the X' direction from the center toward the periphery, the X' direction being exactly opposite to the X direction.

4. The ophthalmic lens according to claim 2 or 3,
wherein a cylinder power of the near-vision portion is equal to a cylinder power of the far-vision portion.

5. The ophthalmic lens according to any one of claims 1 to 4,
wherein the ophthalmic lens is a contact lens.

6. The ophthalmic lens according to any one of claims 1 to 4,
wherein the ophthalmic lens is an intraocular lens.

7. A method of designing an ophthalmic lens including an optical portion that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens further including a front surface that is arranged on an object side and a rear surface that is opposed to the front surface,
wherein the front surface is a composite surface including a progressive surface and a toric surface, and
a cylinder power of the near-vision portion is made equal to a cylinder power of the far-vision portion.

8. The method of designing an ophthalmic lens according to claim 7,
wherein the intermediate portion includes a portion A1 in which a power is strengthened and thereafter weakened when the lens is viewed in an X direction from the center toward the periphery, and the intermediate portion includes a portion A1' in which the power is strengthened and thereafter weakened when the lens is viewed in an X' direction from the center toward the periphery, the X' direction being exactly opposite to the X direction, the power being strengthened in the portion A1 and the portion A1' to be stronger than the far-vision power of the far-vision portion that is arranged in the ring shape along the outer edge of the intermediate portion or the near-vision power of the near-vision portion that is arranged in the ring shape along the outer edge of the intermediate portion.

9. The method of designing an ophthalmic lens according to claim 8,
wherein the near-vision portion or the far-vision portion that is arranged at the center of the optical portion includes a portion A2 in which a power is strengthened and thereafter weakened when the lens is viewed in the X direction from the center toward the periphery, and the near-vision portion or the far-vision portion that is arranged at the center of the optical portion includes a portion A2' in which the power is strengthened and thereafter weakened when the lens is viewed in the X' direction from the center toward the periphery, the X' direction being exactly opposite to the X direction.

10. The method of designing an ophthalmic lens according to claim 8 or 9,
wherein a cylinder power of the near-vision portion is equal to a cylinder power of the far-vision portion.

11. The method of designing the ophthalmic lens according to any one of claims 7 to 10,
wherein the ophthalmic lens is a contact lens.

12. The method of designing an ophthalmic lens according to any one of claims 7 to 10,
wherein the ophthalmic lens is an intraocular lens.

13. A method of manufacturing an ophthalmic lens, comprising:
a design step of designing an ophthalmic lens using the method of designing an ophthalmic lens according to any one of claims 7 to 12; and
a processing step of manufacturing the designed ophthalmic lens using a processing device.

14. An ophthalmic lens set comprising a plurality of ophthalmic lenses that each include an optical portion that includes a near-vision portion having a near-vision power that corresponds to a near distance, a far-vision portion having a far-vision power that corresponds to a distance that is farther than the near distance, and an annular intermediate portion that connects the near-vision portion and the far-vision portion to each other, a central optical portion which is the near-vision portion or the far-vision portion being arranged at the center and an outer optical portion which is the far-vision portion or the near-vision portion that is not arranged at the center being arranged in a ring shape along an outer edge of the intermediate portion, the ophthalmic lens further including a front surface that is arranged on an object side and a rear surface that is opposed to the front surface,
wherein the front surface is a composite surface including a progressive surface and a toric surface, and
a cylinder power of the near-vision portion is equal to a cylinder power of the far-vision portion.
